**Europäisches Patentamt**

**European Patent Office**

**Office européen des brevets**

(19)

(11) Publication number : **0 401 884 B1**

## (12) EUROPEAN PATENT SPECIFICATION

(45) Date of publication of patent specification :
28.07.93 Bulletin 93/30

(51) Int. Cl.⁵ : **C02F 1/32**

(21) Application number : **90201172.5**

(22) Date of filing : **09.05.90**

(54) **Reactor for photooxidations in an aqueous environment.**

(30) Priority : **11.05.89 IT 2044689**
**30.06.89 IT 2103889**

(43) Date of publication of application :
**12.12.90 Bulletin 90/50**

(45) Publication of the grant of the patent :
**28.07.93 Bulletin 93/30**

(84) Designated Contracting States :
**AT BE CH DE DK ES FR GB GR LI LU NL SE**

(56) References cited :
**EP-A- 0 306 301**
**DE-A- 3 245 811**
**WATER RESEARCH, vol. 20, no. 5, May 1986,**
**Pergamon Press Ltd., Exeter, Devon (GB);**
**R.W.MATTHEWS, pp. 569-578**
**PATENT ABSTRACTS OF JAPAN, vol. 12, no.**
**258 (C-513), 20 July 1988**

(73) Proprietor : **ENIRICERCHE S.p.A.**
**Corso Venezia 16**
**I-20121 Milan (IT)**

(72) Inventor : **Miano, Fausto**
**Via Trapani 6**
**I-94100 Enna (IT)**
Inventor : **Borgarello, Enrico**
**Corso Grosseto 274**
**I-10148 Turin (IT)**

(74) Representative : **Roggero, Sergio et al**
**Ing. Barzanò & Zanardo Milano S.p.A. Via**
**Borgonuovo 10**
**I-20121 Milano (IT)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 401 884 B1

## Description

The present invention relates to a method for performing processes of heterogeneous photocatalysis, which process optimizes the exploitation of the radiations emitted by the light source and can be applied to large reaction volumes. Thanks to these features, the present method is highly interesting in particular for carrying out processes of heterogeneous photocatalysis on an industrial scale.

By now, a large number of photocatalytic processes in heterogeneous phase have been described in pertinent technical literature.

Such processes are characterized by the use as the catalyst, in aqueous media, of a semiconductor, such as, e.g., $TiO_2$, on whose surface the substance is adsorbed, which has to be transformed.

By irradiation with photons having a sufficiently high energy, the promotion of an electron from the "valency band" to the "conduction band" of semiconductor is carried out.

In that way, a separation of charges, denominated "electron-hole pair" is accomplished, which migrates to the interface between the liquid phase and the semiconductor, where it is capable of causing redox reactions on the adsorbed compounds.

Many chemical substances exist, which when are individually taken and are submitted to such a treatment undergo transformations: e.g., CN- and $SO_3^{2-}$, which are oxidated ([Bard, J. Phys. Chem. 1977-81 (1984)], organic acids, which are decarboxylated (Bard, J.A.C.S. 1978 100:2239), hydrocarbon compounds, which undergo oxidation, atrazine, which is transformed into cyanuric acid.

Some substances in particular, under the hereinabove disclosed conditions, are completely mineralized, i.e., their skeleton of carbon atoms is completely converted into carbon dioxide. For example, aliphatic and aromatic halides, phenols and chlorophenols can be completely mineralized by means of heterogeneous photocatalysis [Pelizzetti, Serpone, Borgarello, Photocatalysis and Environment 467-497 M. Schiavello (Ed.) 1988]

Therefore, the great importance is evident, which such a type of reaction can have in the fight against the pollution of drinkwater and in general of aquatic environment.

In this regard, the decay due to the mineralization of, besides the hereinabove mentioned compounds, dichlorobenzenes, chloronitrobenzenes, polychlorobiphenyls and polychlorodioxines (Barbeni Chemosphere 15: 1913 1986) is known.

Such descriptions relate to processes accomplished on the laboratory scale, whilst their application on the industrial scale involves considerable problems as regards the equipment and the possibility of application to complex polluting mixtures.

As regards the first aspect, photochemical reactors of the "submerged-lamp" type are known. They substantially consists of a conventional chemical reactor in which a suitable lamp, as the source of photons, is inserted through the cover and is dipped in the reaction mixture, which contains the substance to be demolished and the catalyst.

This type of reactor suffers from the following drawbacks:

(a) A cooling jacket is necessary in order to prevent the lamp from overheating in case a high-pressure mercury-vapour or high-pressure xenon-vapour lamp is used.

Inside this jacket the precipitation of inorganic salts and the formation of seaweeds can occur. Thus, opaque regions are formed which, together with the effect of characteristic absorption of the glass the same jacket is made from, act as a shield and absorb a portion of photons, which are hence lost.

(b) The reactor is so designed, as to be able to contain the whole volume of the reaction mixture, and therefore is not so dimensioned as to match the geometry of the lamp, as well as of the irradiation region.

In fact, it is well-known that the distance inside the reaction mixture the photons are capable of traveling over before losing their energy is very short, and therefore the farthest away regions of the reactor are not reached by a large enough amount of radiations.

This latter drawback cannot be overcome even if stirring in provided.

Other types of reactors which can be used on an industrial scale are the annular reactor, the "Tarkoy-Campana" reactor and the "Braun-De Meijere reactor".

The first one is formed by two coaxial cylinders which bound the reaction region, with the lamp being arranged on the symmetry axis. In this case too, a cooling jacket is provided between the lamp and the reaction mixture.

In "Tarkoy-Campana" reactor, disclosed in US-3,628,010, the geometry of the reaction region is optimized relatively to the dimensions and the length of the lamp. The same advantages are obtained by using the "Braun-De Meijere" reactor (A.M. Braun "Technologie photochimique" Presses Politechniques Romandes, Lausanne, 1986).

These last two types of photochemical reactor are very efficient, but in the particular case of photocatalytic reactions in heterogeneous phase they are difficult to be used, owing to the presence of suspended catalyst

2

particles in the reaction mixture.

The present Applicant has found now a reactor for heterogeneous photocatalysis, which is characterized by the absence of the cooling jacket between the lamp and the reaction mixture. The reactor consists of a hollow space, whose inner wall is the wall of a lamp which emits at least one fraction of its radiations with a wavelength shorter than 400 nm, and preferably comprised within the range of from 320 to 370 nm.

Temperature control is obtained by causing the reaction mixture to continuously circulate through said hollow space.

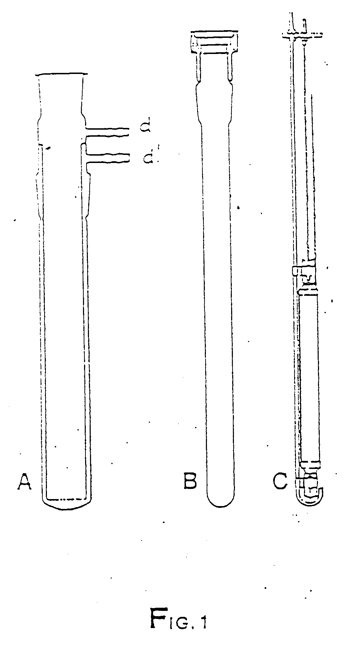

In Figure 1 the components of the reactor, and the lamp are shown: A is the outer wall of the reactor, and B is the container of the lamp C.

By sliding B into A and connecting them by means of a conical fitting, the jacket is formed, inside which the reaction mixture circulates; d and d' indicate the inlet and the outlet ways for the reaction mixture.

When such a type of reactor is used, the loss of photons can be avoided, which is due to absorption both by the opaque regions formed by the precipitation of inorganic salts, or owing to the growth of seaweeds, as well as to absorption by the glass walls normally existing between the lamp and the reaction mixture.

Furthermore, with this reactor large amounts of solutions can be processed, without the risk -- as it occurs in the reactor of submerged-lamp type -- that reactor regions, and therefore regions of the reaction mixture, are too far away from the lamp, and therefore cannot be reached by the light emitted by the lamp.

The lamps useable in the "jacket" reactor which is the object of the present invention are high-pressure mercury-vapour or high-pressure xenon-vapour arc lamps.

The jacket reactor has a width of not more than 5 cm, and preferably not higher than 2.5 cm. It substantially consists of a sheath inside which the reaction mixture flows.

Inside this sheath baffles can be installed, which generate an obliged path for the reaction mixture, increasing the stay time thereof inside the interior of the reactor, so as to favour the interaction of said reaction mixture with the photons generated by the lamp.

The reaction mixture is caused to flow through this sheath with a flowrate which is computed on the basis of the power of the lamp, with said power being expressed as watts, and therefore obtaining how much aqueous mixture is necessary in order to exchange such a heat amount (it is therefore assumed that the coefficient of heat exchange of the reaction mixture is the same as of water). On the basis of the results which one wishes to obtain, a reaction mixture will be exposed to irradiation by being caused to flow a plurality of times through the jacket reactor. This result can be accomplished by connecting with the reactor a tank containing the reaction mixture and equipped with a pump which secures a continuous recycle of the reaction mixture between the reactor and the tank.

The same results as of the preceding "batchwise" system can be obtained by operating in continuous with a long enough lamp, or with a plurality of lamps arranged in cascade.

A set of tests were carried out using for exemplifying purposes a jacket reactor of 100 ml of volume, and a mercury-vapour lamp with a specific power of 40 W/cm and an useful length of 3 cm.

100 ml of a mixture containing 50 ppm of o-nitrophenol and 1 g of $TiO_2$/litre dispersed in water were charged to this reactor.

This is a reference test, in which the volume of solution submitted to the treatment of photocatalysis was equal to the volume of the reactor, and therefore, inasmuch as a circulation of the reaction mixture did not take place, an external cooling had to be applied.

The data resulting from this test indicates the better reaction yield which can be obtained when the cooling sheath is eliminated.

After 60 minutes of irradiation, the solution resulted to contain 14.6 ppm of unreacted product, whilst the residual amount was decomposed up to mineralization.

A test under similar conditions was carried out on 500 ml of the above mixture.

In this case, the reactor of 100 ml of capacity was connected with a tank which contained the residual portion of the reaction mixture.

The circulation of the reaction mixture between the reactor and the tank was obtained by means of a pump, and in this way the cooling of the lamp was secured.

After 60 minutes, the concentration was decreased from 50 ppm to 16 ppm. In other terms, it was possible to process a volume which is 5 times as large as the volume treated in the reference test, with the decomposition rate remaining constant. In order to reach the threshold of loss of efficiency of this reactor, a volume 10 times as large as the volume treated in the reference test had to be processed, In fact, when 1 litre of reaction mixture containing 50 ppm of ortho-nitrophenol and 1 g of $TiO_2$/litre was submitted to irradiation, after 60 minutes a concentration of 24 ppm was obtained.

The results which can be obtained with the "jacket" reactor according to the present invention considerably improve if into the reaction mixture air is injected.

As the reference test, 200 ml of a mixture containing 50 ppm of o-nitrophenol and 1 g of TiO$_2$/litre was submitted to a treatment of heterogeneous photocatalysis inside a jacket reactor of 200 ml of volume, equipped with a lamp with a specific power of 40 W/cm and an useful length of 3 cm. Air was injected into the interior of the reactor. Also in this case, inasmuch as the reaction mixture was not circulated, an external cooling was applied. With this test, the results were evaluated, which can be obtained with a "jacket" reactor, i.e., without the hollow space of the cooling jacket, when into the reaction mixture air is injected.

After 30 minutes of irradiation, only 9.2 ppm of o-nitrophenol had remained, which decreased down to less than 1 ppm after 60 minutes.

The volume of the reaction mixture to be processed was then increased to a 2.5 times larger volume. 500 ml of this reaction mixture was submitted to irradiation inside the same reactor, which this time was connected with a suitable tank and a pump in order to secure the continuous circulation of the reaction mixture through the system.

Air can be injected into the reaction circuit indifferently at reactor level, and at tank level.

From this second test, the same results were obtained again, which were obtained from the reference test: after 30 minutes, the concentration decreased down to 8.8 ppm, and after 60 minutes it was lower than 1 ppm.

For comparison purposes, 1.6 l of the same solution was processed in the presence of 1 g of TiO$_2$/litre and under a stream of air, in a reactor of submerged-lamp type, equipped with a 1000-W lamp, therefore extremely powereful, whose cooling was secured by means of a suitable cooling jacket.

After 60 minutes of treatment, the solution resulted to contain still 25 ppm of o-nitrophenol.

The same result was obtained when a solution of 50 ppm of chlorophenol was submitted to irradiation under similar conditions.

This worsening observed in the results is to be attributed both to the fact that the cooling jacket acts like a shield for the photons emitted by the lamp, and the comparatively large reaction volume, the latter being such as not to make it possible to irradiate the entire mixture evenly.

In order to overcome the drawbacks aforementioned, the present invention provides a method for catalytically oxidizing by irradiation a polluted aqueous mixture which contains Ti-dioxide dispersed therein, comprising the steps of:

causing said polluted aqueous mixture to flow through an irradiating reactor consisting of a tubular outer container equipped with laterally and superposedly positioned inlet and outlet tubes (d, d') for circulating said polluted aqueous mixture, said tubular outer container housing an inner tubular lamp-casing which encases an irradiating lamp, no cooling jacket being interposed between said tubular outer container and said inner tubular lamp-casing, the maximum radial distance between said tubular outer container and said inner tubular lamp-casing being 5 cm, and

irradiating the polluted aqueous mixture, flowing through the interspace between said tubular outer container and said inner tubular lamp-casing, with an irradiating lamp whose radiation-emitting spectrum comprises radiations having a maximum wavelength of 400 nm.

The other aspect of the present invention is, therefore, an apparatus for carrying out the method referred to above, which consists of a tubular outer container equipped with laterally and superposedly positioned inlet and outlet tubes for circulating said polluted aqueous mixture, said tubular outer container housing an inner tubular lamp-casing which encases an irradiating lamp, no cooling jacket being interposed between said tubular outer container and said inner tubular lamp-casing, the maximum radial distance between said tubular outer container and said inner tubular lamp-casing being 5 cm. Consistently with the results aimed at, the photocatalytic treatment time can be extended by causing the polluted mixture to flow may a time through the jacketless reactor mentioned above.

The result is achieved by connecting the reactor to a tank and circulating the polluted mixture through the reactor by a pump: as an alternative, the tank can be dispensed with and the operation may be continuous either by adopting a sufficiently long lamp, or a plurality of serially connected lamps. If so, the reactor shall be appropriately sized.

The method and the apparatus outlined in the foregoing are such as to provide the following advantages, namely:

(a) the radiations emitted by the lamp are better exploited, without photons getting lost owing to the absorption by a cooling jacket; therefore, the reaction yield is maximized.

The temperature of the lamp is controlled by the same reaction mixture, which is made circulate in continuous.

(b) This instant system can be applied to large reaction volumes, with the treatment yields being kept constant. With the "submerged-lamp" reactors according to the prior art, an increase in volume causes a decrease in reaction yield.

The method according to the present invention can be advantageously used in the field of the fight against environmental pollution, in order to purify drinkwaters and natural waters from both organic and inorganic pollutants, such as fungicides, herbicides, cyanides, and so forth.

Such a method can be applied as well to complex mixtures of organic and inorganic compounds, such as those mixtures which are found in waste waters from chemical factories, zootechnical factories, and so forth.

In this regard, a complex mixture of organic compounds deriving from the waste liquors from a chemical factory was submitted to a process of heterogeneous photocatalysis carried out inside a "jacket" reactor. Before being submitted to said treatment, this mixture was characterized by a COD (Chemical Oxygen Demand) of 54 mg of $O_2$ per litre. After a 5-hours-long treatment of 1000 ml of such a mixture in the presence of 1 g of $TiO_2$/litre, and with a lamp with a specific power of 40 W/cm and an useful length of 3 cm, inside a jacket reactor of 100 ml of capacity connected with a suitable tank and equipped with a pump which caused the reaction mixture to continuously circulate, a COD lower than 10 mg/l of $O_2$ was measured.

Equally satisfactory results were obtained when the waste liquors coming from a pig-breeding factory were submitted to a photocatalytic treatement.

In particular, 500 ml of a mixture with a COD of 320 mg of $O_2$/l were irradiated by means of two lamps in cascade, each of which has a specific power of 40 W/cm and a length of 3 cm. After 4 hours of irradiation, COD value resulted to be of 84 mg of $O_2$/litre.

The method according to the present invention can hence be indifferently applied to solutions containing one compound only, or to complex mixtures of organic or inorganic compounds.

As the catalyst in the process according to the present invention $TiO_2$ powder is used which, once added to the polluted solutions, forms colloidal suspensions or solutions; or $TiO_2$ is used in the form of microspheres.

In case $TiO_2$ powder is used, the catalyst is recovered at the end of the process by decantation or centrifugation. The resulting liquors contain less than 1 mg of $TiO_2$/litre, can be disposed of to the environment, but they are not drinkwater.

Therefore, a preferred aspect of the instant invention is submitting aqueous solutions of pollutant substances to a photocatalytic treatment inside the hereinabove disclosed reactor, using microspheres of $TiO_2$ of diameter comprised within the range of from 0.1 to 5.0 mm as the catalyst, under such flowing conditions as to generate a fluidized bed of catalyst. In such a way, the catalyst remains always inside the reactor and after the photocatalytic treatment no further steps of purification of the resulting liquors are necessary any longer in order to recover the catalyst and render said liquors pure enough in order it to be disposed of to the environment.

Furthermore, owing to the fact that the catalyst remains confined inside the reactor, the probability that said catalyst is reached by the light radiations increases, with the probability that the pollutant substances adsorbed on said catalyst surface are demolished consequently increasing.

Furthermore, the microspheres of $TiO_2$ show a mechanical strength by far higher than of the catalysts based on supported titanium dioxide, e.g., on glass.

In fact, these latter, owing to the rubbing they undergo, tend to crumble and to release $TiO_2$ powder which, at the end of the process, causes the above mentioned separation problems.

It should be finally stressed that the useful life time of this microsphere catalyst is very high: in fact, possible substances tending to accumulate on $TiO_2$ surface can be removed by means of a sufficient irradiation, and do not modify its reactivity.

Therefore, a preferred aspect of the present invention is a method for the photocatalytic treatment, in the presence of titanium dioxide, of aqueous mixtures of pollutants, which consists in submitting said mixtures to irradiation while they are caused to circulate inside a reactor formed by a hollow space of thickness of not more than 5 cm, whose inner wall is the wall of a lamp emitting at least a fraction of its radiations with a wavelength shorter than 400 nm, which method is characterized in that according to it $TiO_2$ in the form of microspheres with a diameter comprised within the range of from 0.1 to 5.0 mm is used, which microspheres are kept moved inside the interior of the reactor by the flowing stream of the reactant mixture, but without being removed.

$TiO_2$ microspheres which can be used in the process according to the present invention are those prepared according to GB-A-2.112.764 by mixing a titanium halide and an alcohol in order to yield an alcoholic solution of the corresponding organometallic titanium compound, thickening said solution by adding an organic polymer to it, then subdividing it by means of an atomizer into liquid droplets which are immediately solidified into gel particles by contact with an alcoholic solution of alkali; the gel particles are then dried or calcined.

$TiO_2$ prepared according to GB-A-2.112.764 has a specific surface area of 150 m²/g vs. the specific surface area of 50 m²/g of $TiO_2$ powder and of 0.5 m²/g of glass supported $TiO_2$: its use in a photocatalytic process favours a better contact between the catalyst and the mixture of pollutant substances.

The method according to the present invention, in its preferred aspect according to which the catalyst is constituted by $TiO_2$ microspheres, can be carried out either continuously or batchwise.

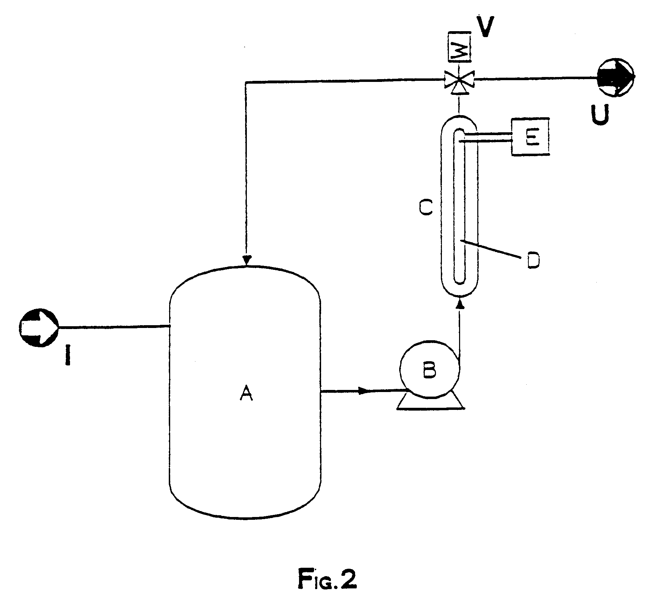

A possible relization of the first case is shown in Figure 2: the reaction mixture is fed through the inlet I to the tank A, and from this latter it is charged to the reactor C by means of the pump B. Inside the reactor the reaction mixture undergoes the irradiation, in the presence of the microspheres of titanium dioxide, by the lamp D, powered by the electrical generator E. From the outlet U, controlled by the electrovalve V, an aliquot of the reaction mixture at the desired concentration is discharged, with the remainder portion of said reaction mixture being recycled to the tank.

The batchwise process can be accomplished according to a process scheme analogous to the preceding one, with the difference that all of the mixture to be purified is contained inside the tank A and is circulated through the reactor C so as to be submitted a plurality of times to the irradiation, until the desired concentration of pollutant substances is reached. By operating according to the continuous mode, the following advantages are achieved:

1) the dead times required by the discharge and feed restoration are eliminated; and

2) also polluting mixtures with a so high pollutant concentration as not to meet the optimum conditions to have mineralization can be used. In fact, in the steady state, the volume of mixture of pollutant substances entering the tank at each time is diluted by the already contained mixture, yielding such a concentration that, by means of one pass through the reactor, the desired end concentration is reached.

In the following some Examples of application of the method according to the present invention in its preferred aspect in which the catalyst is constituted by microspheres of $TiO_2$ are reported.

Example 1

500 ml of an aqueous mixture containing 50 ppm of o-nitrophenol is submitted to photooxidation in the presence of 10 g of $TiO_2$ microspheres inside a jacket-reactor similar to the hereinabove disclosed reactors, of 2.5 mm of thickness, equipped with a high-pressure mercury-vapours lamp of 125 W and installed in a batchwise-operating facility.

The mixture is first circulated 15 minutes in the darkness, so that the surface of the catalyst gets saturated by the pollutant substances. A concentration of the solution of 37 ppm is thus obtained, and then the irradiation is carried out.

In the following table the obtained results are reported:

| Time (minutes) | Concentration (ppm) |
|---|---|
| 0 | 37 |
| 30 | 11 |
| 60 | 4 |

At the end of the reaction the amount of produced $CO_2$ was determined, and resulted to be of 1 mmol.

Example 2

500 ml of an aqueous mixture of o-nitrophenol is treated in the same way as disclosed in Example 1 inside a jacket-reactor of 5.0 mm of thickness, equipped with a low-pressure mercury-vapours lamp of 40 W, in the presence of 40 g of $TiO_2$ microspheres.

The results obained are reported In the following table:

| Time (minutes) | Concentration (ppm) |
|---|---|
| 0 | 37 |
| 30 | 29 |
| 60 | 22 |
| 120 | 15 |
| 240 | 5 |

Example 3

500 ml of an aqueous mixture deriving from the waste liquors from a pig-breeding factory is submitted to photooxidation in the presence of 10 g of $TiO_2$ as microspheres inside a reactor like the one disclosed in Example 1, equipped with two high-pressure mercury-vapours lamps of 120 W. Before undergoing the treatment, the mixture showed a values of COD (Chemical Oxygen Demand, code IRSA E007) of 300 mg of $O_2$/litre. After a 4-hours treatment COD value has decreased down to 157 mg of $O_2$/litre, and after 6 hours it was of 68 mg of $O_2$/litre.

Example 4

1000 ml of an aqueous mixture deriving from the waste liquors from a chemical industry was submitted to photooxidation under the same conditions ad disclosed in the preceding Example. The so obtained results are reported in the following Table:

| Time (hours) | Concentration (mg/l of $O_2$) |
|---|---|
| 0 | 47 |
| 6 | <10 |

Example 5

500 ml of an aromatics-containing aqueous mixture was treated in the same way as disclosed in Example 2.

The results obtained are reported in the following Table:

| Time (hours) | Concentration (mg/l of $O_2$) |
|---|---|
| 0 | 90 |
| 2 | 72 |
| 8 | 35 |

Example 6

In a facility like the one depicted in Figure 2, a solution containing 50 ppm of o-nitrophenol is fed to the tank A at a flowrate of 100 ml/hour.

The tank has a volume of 1000 ml, the reactor has a thickness of 5 mm and the lamp, a low-pressure mercury-vapour one, has a power of 40 W.

From the outlet U 100 ml/hour of treated solution containing 9 ppm of polluting substance is discharged.

**Claims**

1. Method for catalytically oxidizing by irradiation a polluted aqueous mixture which contains Ti-dioxide dispersed therein, comprising the steps of:

causing said polluted aqueous mixture to flow through an irradiating reactor consisting of a tubular outer container (A) equipped with laterally and superposedly positioned inlet and outlet tubes (d, d') for circulating said polluted aqueous mixture, said tubular outer container (A) housing an inner tubular lamp-casing (B) which encases an irradiating lamp (C), no cooling jacket being interposed between said tubular outer container (A) and said inner tubular lamp-casing (B), the maximum radial distance between said tubular outer container (A) and said inner tubular lamp-casing (B) being 5 cm, and

irradiating the polluted aqueous mixture, flowing through the interspace between said tubular outer container (A) and said inner tubular lamp-casing (B), with an irradiating lamp whose radiation-emitting spectrum comprises radiations having a maximum wavelength of 400 nm.

2. Method according to Claim 1, wherein the wavelength of the radiations emitted by said irradiating lamp is of from 380 nm to 320 nm.

3. Method according to Claim 1, wherein said irradiating lamp is selected from the high-pressure mercury-vapour lamps and the xenon-vapour arc lamps.

4. Method according to Claim 1, wherein said Ti-oxide is selected from powdered Ti-dioxide and Ti-dioxide microspheres.

5. Method according to Claim 4, wherein the Ti-dioxide microspheres have an outside diameter of from 0,1 mm to 5,0 mm.

6. Method according to Claim 4, wherein the Ti-dioxide microspheres have a specific superficial area of 150 $m^2/g$.

7. Method according to Claim 1, further comprising the step of aërating said aqueous polluted mixture with an oxidizing gaseous medium, selected from air and oxygen, preliminarily to the irradiation thereof.

8. Apparatus for carrying out the process as defined in Claim 1, characterized in that it consists of a tubular outer container (A) equipped with laterally and superposedly positioned inlet and outlet tubes (d, d') for circulating said polluted aqueous mixture, said tubular outer container (A) housing an inner tubular lamp-casing (B) which encases an irradiating lamp (C), no cooling jacket being interposed between said tubular outer container (A) and said inner tubular lamp-casing (B), the maximum radial distance between said tubular outer container (A) and said inner tubular lamp-casing (B) being 5 cm.

## Patentansprüche

1. Verfahren zum katalytischen Oxidieren eines verunreinigten wäßrigen Gemisches mit einem Gehalt an darin dispergiertem Titandioxid durch Bestrahlung, anfassend die folgenden Stufen:
    Strömenlassen dieses verunreinigten wäßrigen Gemisches durch einen Bestrahlungsreaktor, der aus einem rohrförmigen Außenbehälter (A) besteht, der mit seitlich und übereinander angeordneten Einlaß- und Auslaßrohren (d, d') zum Zirkulieren des verunreinigten wäßrigen Gemisches ausgerüstet ist, welcher rohrförmige Außenbehälter (A) eine innere rohrförmige Lampenhülle (B) aufnimmt, die eine Bestrahlungslampe (C) umschließt, wobei zwischen dem rohrförmigen Außenbehälter (A) und der inneren rohrförmigen Lampenhülle (B) kein Kühlmantel zwischengeschaltet ist und wobei der maximale radiale Abstand zwischen dem rohrförmigen Außenbehälter (A) und der inneren rohrförmigen Lampenhülle (B) 5 cm beträgt, und
    Bestrahlen des durch den Zwischenraum zwischen dem rohrförmigen Außenbehälter (A) und der inneren rohrförmigen Lampenhülle (B) strömenden verunreinigten wäßrigen Gemisches mit einer Bestrahlungslampe, deren strahlungsemmitierendes Spektrum Strahlen mit einer maximalen Wellenlänge von 400 nm umfaßt.

2. Verfahren nach Anspruch 1, worin die Wellenlänge der von der Bestrahlungslampe emittierten Strahlen von 380 nm bis 320 nm beträgt.

3. Verfahren nach Anspruch. 1, worin die Bestrahlungslampe unter Hochdruck-Quecksilberdampflampen und Xenondampf-Bogenlampen ausgewählt ist.

4. Verfahren nach Anspruch 1, worin das Titanoxid unter pulverförmigem Titandioxid und Titandioxidmikrokügelchen ausgewählt ist.

5. Verfahren nach Anspruch 4, worin die Titandioxidmikrokügelchen einen Außendurchmesser von 0,1 mm bis 5,0 mm aufweisen.

6. Verfahren nach Anspruch 4, worin die Titandioxidmikrokügelchen eine spezifische Oberfläche von 150 $m^2/g$ aufweisen.

7. Verfahren nach Anspruch 1, welches weiterhin die Stufe des Belüftens des wäßrigen verunreinigten Gemisches mit einem unter Luft und Sauerstoff ausgewählten oxidierenden gasförmigen Medium vor dem Bestrahlen des Gemisches umfaßt.

8. Vorrichtung zur Ausführung des Verfahrens nach Anspruch 1, dadurch gekennzeichnet, daß sie aus einem rohrförmigen Außenbehälter (A), der mit seitlich und übereinander angeordneten Einlaß- und Auslaßrohren (d, d') zum Zirkulieren des verunreinigten wäßrigen Gemisches ausgerüstet ist, besteht, welcher rohrförmige Außenbehälter (A) eine innere rohrförmige Lampenhülle (B) aufnimmt, die eine Bestrahlungslampe (C) umschließt, wobei zwischen diesem rohrförmigen Außenbehälter (A) und dieser inneren rohrförmigen Lampenhülle (B) kein Kühlmantel zwischengeschaltet ist, und wobei der maximale Radialabstand zwischen dem rohrförmigen Außenbehälter (A) und der inneren rohrförmigen Lampenhülle (B) 5 cm beträgt.

## Revendications

1. Procédé d'oxydation catalytique par irradiation d'un mélange aqueux pollué qui contient du dioxyde de titane dispersé dedans, comportant les étapes consistant à :

faire circuler ledit mélange aqueux pollué dans un réacteur d'irradiation constitué d'un récipient extérieur tubulaire (A) muni de tubes d'entrée et de sortie (d, d'), disposés latéralement et superposés, destinés au passage dudit mélange aqueux pollué, ledit récipient extérieur tubulaire (A) abritant une gaine intérieure tubulaire (B) pour lampe, qui enveloppe une lampe d'irradiation (C), aucune chemise de réfrigération n'étant disposée entre ledit récipient extérieur tubulaire (A) et ladite gaine intérieure tubulaire (B) pour lampe, la distance radiale maximale entre ledit récipient extérieur tubulaire (A) et ladite gaine intérieure tubulaire (B) pour lampe valant 5 cm, et

l'irradiation du mélange aqueux pollué circulant dans l'espace situé entre ledit récipient extérieur tubulaire (A) et ladite gaine intérieure tubulaire (B) pour lampe, à l'aide d'une lampe d'irradiation dont le spectre d'émission de rayonnement comprend un rayonnement ayant une longueur d'onde d'au maximum 400 nm.

2. Procédé conforme à la revendication 1, dans lequel les longueurs d'onde du rayonnement émis par ladite lampe d'irradiation vont de 380 nm à 320 nm.

3. Procédé conforme à la revendication 1, dans lequel ladite lampe d'irradiation est choisie parmi les lampes à vapeur de mercure sous haute pression et les lampes à arc à vapeur de xénon.

4. Procédé conforme à la revendication 1, dans lequel ledit oxyde de titane est choisi parmi du dioxyde de titane en poudre et des microsphères de dioxyde de titane.

5. Procédé conforme à la revendication 4, dans lequel le diamètre extérieur des microsphères de dioxyde de titane vaut de 0,1 mm à 5,0 mm.

6. Procédé conforme à la revendication 4, dans lequel l'aire spécifique des microsphères de dioxyde de titane vaut 150 $m^2$/g.

7. Procédé conforme à la revendication 1, qui comporte en outre l'étape consistant à aérer ledit mélange aqueux pollué à l'aide d'un gaz oxydant choisi parmi l'air et l'oxygène, avant de l'irradier.

8. Appareil destiné à la réalisation du procédé conforme à la revendication 1, caractérisé en ce qu'il est constitué d'un récipient extérieur tubulaire (A) muni de tubes d'entrée et de sortie (d, d'), disposés latéralement et superposés, destinés au passage dudit mélange aqueux pollué, ledit récipient extérieur tubulaire (A) abritant une gaine intérieure tubulaire (B) pour lampe, qui enveloppe une lampe d'irradiation (C), aucune chemise de réfrigération n'étant disposée entre ledit récipient extérieur tubulaire (A) et ladite gaine intérieure tubulaire (B) pour lampe, la distance radiale maximale entre ledit récipient extérieur tubulaire (A) et ladite gaine intérieure tubulaire (B) pour lampe valant 5 cm.

F~IG.~1~

Fig.2